(19) Europäisches Patentamt
European Patent Office
Office européen des brevets

(11) Veröffentlichungsnummer: **0 073 464**
**B1**

# (12) EUROPÄISCHE PATENTSCHRIFT

(45) Veröffentlichungstag der Patentschrift: **02.05.85**

(21) Anmeldenummer: **82107781.5**

(22) Anmeldetag: **25.08.82**

(51) Int. Cl.⁴: **C 07 C 63/40**, C 07 C 51/31, C 07 C 46/04, C 07 C 50/22, C 07 C 79/46

## (54) Verfahren zur Herstellung von Naphthalintetracarbonsäure-1,4,5,8.

(30) Priorität: **28.08.81 DE 3134131**
**28.08.81 DE 3134133**

(43) Veröffentlichungstag der Anmeldung: **09.03.83 Patentblatt 83/10**

(45) Bekanntmachung des Hinweises auf die Patenterteilung: **02.05.85 Patentblatt 85/18**

(84) Benannte Vertragsstaaten: **CH DE FR GB LI**

(56) Entgegenhaltungen:
**DE - A - 2 358 481**
**GB - A - 853 369**
**US - A - 2 786 076**

(73) Patentinhaber: **HOECHST AKTIENGESELLSCHAFT, Postfach 80 03 20, D-6230 Frankfurt am Main 80 (DE)**

(72) Erfinder: **Röhrscheid, Freimund, Dr., Amselweg 24, D-6233 Kelkheim (Taunus) (DE)**
Erfinder: **Riemenschneider, Wilhelm, Dr., Loreleistrasse 45, D-6230 Frankfurt am Main 80 (DE)**

EP 0 073 464 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Naphthalintetracarbonsäure-1,4,5,8 – im folgenden kurz Tetrasäure genannt – ist ein wichtiges Vorprodukt für Farbstoffe, die sich durch besonders hohe Lichtechtheit auszeichnen. So lassen sich beispielsweise durch Umsetzung von Tetrasäure mit aromatischen Diaminen brillante und lichtechte Farbstoffe herstellen.

Dementsprechend sind auch Herstellungsverfahren für Tetrasäure bekannt, z.B. aus Fierz-David, „Grundlegende Operationen der Farbenchemie", 8. Aufl., Wien 1952, S. 219-221. Als Ausgangsmaterial wird fast ausnahmslos Pyren verwendet, das in einem mehrstufigen Prozess mit Halogenen und Schwefeltrioxid zu Tetrasäure oxidiert wird. Diese Verfahren haben jedoch den Nachteil, dass sie in konzentrierter Schwefelsäure als Lösungsmittel ausgeführt werden. Daraus resultiert – abgesehen von verfahrenstechnischen Schwierigkeiten – eine starke Umweltproblematik, die insbesondere in der Beseitigung der ganz erheblichen Schwefelsäuremengen besteht.

Es wurde deshalb bereits vorgeschlagen, die Oxidation des Pyrens in essigsaurer Lösung mit Sauerstoff und Ozon in Gegenwart von Katalysatoren wie Mangan- und Kobaltsalzen durchzuführen, wobei – gegebenenfalls nach einer zusätzlichen Oxidationsstufe mit Hypochlorit – Naphthalintetracarbonsäure in Ausbeuten bis zu 70% d.Th., bezogen auf umgesetztes Pyren, erhältlich sein soll (SU-PS Nr. 202920). Wie die Nacharbeitung dieses Verfahrens ergeben hat (vgl. Beispiel 1), entsteht dabei die Naphthalintetracarbonsäure-1,4,5,8 jedoch allenfalls in einer Ausbeute von wenigen Prozenten.

Es bestand deshalb nach wie vor das Bedürfnis nach einem verbesserten Verfahren zur Herstellung von Naphthalintetracarbonsäure-1,4,5,8 aus Pyren, welches ohne Verwendung von Schwefelsäure das Produkt in zufriedenstellender Ausbeute liefert.

Überraschenderweise wurde nun gefunden, dass sich dieses Ziel dadurch erreichen lässt, dass man das Pyren in einer ersten Stufe in Gegenwart einer katalytisch wirksamen Metallverbindung in einer niederen Alkancarbonsäure mit einer organischen Perverbindung bzw. mit einem System, in dem unter den Reaktionsbedingungen eine Perverbindung entsteht, zu einer Zwischenverbindung $Z_1$ oxidiert und diese in bekannter Weise mit Hypochlorit in das Alkalisalz der Naphthalintetracarbonsäure überführt. Eine vorteilhafte Verfahrensvariante besteht darin, dass das Zwischenprodukt $Z_1$ vor der Behandlung mit Chlor (Hypochlorit) einer weiteren Oxidation mit Sauerstoff oder Luft in Gegenwart von Salpetersäure und gegebenenfalls einer Vanadinverbindung zu einem Zwischenprodukt $Z_2$ unterworfen wird.

Gefunden wurde ein Verfahren zur Herstellung von Naphthalintetracarbonsäure-1,4,5,8 durch Oxidation von Pyren, das dadurch gekennzeichnet ist, dass man Pyren, gelöst oder suspendiert in einer niederen Alkancarbonsäure, vorzugsweise in essigsaurer Lösung oder Suspension, in Gegenwart einer katalytisch wirksamen Verbindung der Metalle Chrom, Mangan, Eisen, Kobalt, Nickel oder Kupfer mit einer organischen Perverbindung oder mit einem System, in dem unter den Reaktionsbedingungen eine organische Perverbindung entsteht, zu einer Zwischenverbindung $Z_1$ oxidiert und diese in bekannter Weise mit Chlor (Hypochlorit) in wässerig-alkalischer Lösung in das Alkalisalz der Naphthalintetracarbonsäure-1,4,5,8 überführt.

Das erfindungsgemässe Verfahren lässt sich durch das folgende vereinfachte Schema veranschaulichen:

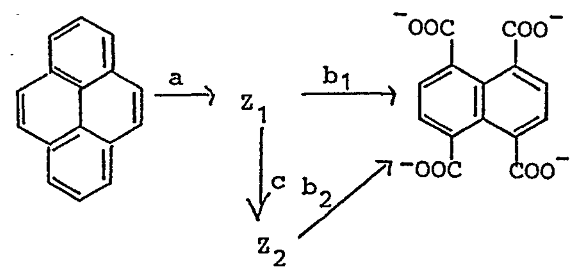


a: Oxidation mit organischer Perverbindung bzw. mit einem System, in dem unter den Reaktionsbedingungen eine organische Perverbindung entsteht.

$b_1$, $b_2$: Oxidation in bekannter Weise mit Chlor (Hypochlorit).

c: Oxidation mit Sauerstoff oder Luft in Gegenwart von Salpetersäure.

Die Zwischenverbindung $Z_1$ besteht aus einem Gemisch, das vorwiegend cis- oder trans-Pyrenchinon enthält. Die Zwischenverbindung $Z_2$ besteht aus einem Gemisch mit einem vorwiegenden Anteil an der Verbindung der allgemeinen Formel

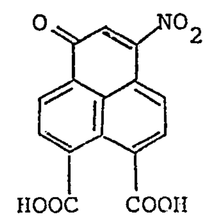

Pyren löst sich in warmer Essigsäure verhältnismässig gut, so dass die Oxidation in homogener Lösung ausgeführt werden kann. Vorteilhaft kann man jedoch beträchtliche Mengen des Lösungsmittels einsparen, wenn man das Pyren zuerst in der gerade notwendigen Menge heisser bzw. siedender Essigsäure löst und dann durch Abkühlung bis zur Oxidationstemperatur unter starkem Rühren eine feine Kristallsuspension erzeugt.

Zu der essigsauren Lösung bzw. Suspension wird die Metallverbindung zugesetzt, die die Aufgabe hat, die für technische Zwecke zu langsam verlaufende Oxidation des Pyrens mit den organischen Perverbindungen zu beschleunigen. Als solche Oxidationskatalysatoren sind Salze der zuvor genannten Metalle bevorzugt. Besonders vorteilhaft sind entsprechende Salze der niederen Alkancarbonsäuren, insbesondere die Acetate, da dadurch keine fremden Anionen in das Reaktionssystem eingeführt werden, wodurch die Aufarbeitung erleichtert wird. Kleine Mengen von fremden

Anionen stören die Reaktion jedoch nicht. Als besonders geeignet erweist sich der Zusatz von Kobalt- und/oder Manganacetat.

Von den Metallverbindungen genügen katalytische Mengen, bevorzugt sind 0,005 bis 0,1 g-Atom je Mol eingesetztes Pyren.

Als Oxidationsmittel werden organische Perverbindungen wie Hydroperoxide oder Percarbonsäuren verwendet. Als Hydroperoxide kommen beispielsweise tert.-Butylhydroperoxid, Cumolhydroperoxid oder Ethylbenzol-1-hydroperoxid (1-Hydroperoxy-1-phenylethan) in Betracht. Als Percarbonsäuren können aliphatische oder aromatische Verbindungen wie Peroxyalkancarbonsäuren oder Perbenzoesäure eingesetzt werden, vorteilhaft verwendet man jedoch die dem Reaktionsmedium entsprechende Percarbonsäure, im Falle der Essigsäure also Peressigsäure.

Hierbei kann anstelle der Percarbonsäure auch ein System dienen, in dem sich unter den Reaktionsbedingungen Percarbonsäuren bilden können. So kann Peressigsäure aus Acetanhydrid und Wasserstoffperoxid präformiert oder durch Zugabe dieser Substanzen während der Reaktion laufend nachgebildet werden. Wegen der Gefahr der Rückbildung von Carbonsäure und Wasserstoffperoxid soll die Percarbonsäurelösung möglichst wenig Wasser enthalten. Wasserstoffperoxid muss aus dem Reaktionsgemisch soweit wie möglich entfernt werden, weil es sonst zu sofortiger heftiger Sauerstoffentwicklung kommt infolge der Reaktion:

$$CH_3CO_2OH + H_2O_2 \xrightarrow{[Co]} CH_3COOH + H_2O + O_2$$

In einer besonders bevorzugten Ausführungsform wird eine Lösung von etwa 20% Peressigsäure in Essigsäure schnell in die Pyrensuspension eingetropft, wobei die feinen Kristalle mit dunkelbrauner Farbe in Lösung gehen. Hierbei wird vorwiegend eine chinoide Zwischenstufe gebildet. Hierfür genügen 3 mol Peressigsäure je Mol Pyren. Wesentlich grössere Mengen an Peressigsäure bewirken keine wesentlich höhere Ausbeute an Tetrasäure.

Auch die Hydroperoxide können gegebenenfalls erst während der Reaktion *in situ* erzeugt werden, beispielsweise durch gleichzeitige Zugabe von Isobuten und Sauerstoff das tert.-Butylhydroperoxid.

Die Oxidationstemperatur liegt zweckmässig im Bereich von 0° C bis zum Siedepunkt des Reaktionsmediums und wird nötigenfalls durch Kühlung eingestellt. Besonders vorteilhaft ist ein Temperaturbereich von etwa 10 bis 60° C.

Geignet zur *in-situ*-Bildung von Perverbindungen sind auch Aldehyde bzw. Ketone, die üblicherweise für Kooxidationen verwendet werden, beispielsweise Methylethylketon oder Cyclohexanon, vorzugsweise jedoch Aldehyde, insbesondere Acetaldehyd, in Verbindung mit Sauerstoff.

Bei der Autooxidation der niedermolekularen Aldehyde entstehen die entsprechenden Carbonsäuren, im Fall des Acetaldehyds Peressigsäure. Es ist vorteilhaft, Essigsäure von vornherein als Lösungsmittel zuzusetzen.

Zur Reaktionsmischung wird als Katalysator eine der definierten Metallverbindungen zugesetzt, wie es oben bereits beschrieben wurde.

Das Molverhältnis Pyren zu Acetaldehyd liegt zweckmässig im Bereich von 1:3 bis 1:30, vorzugsweise von 1:6 bis 1:20. Die Temperatur bei der Kooxidation liegt zweckmässig im Bereich von Raumtemperatur bis zum Siedepunkt des Reaktionsmediums und wird nötigenfalls durch Kühlung eingestellt. Vorzugsweise beträgt sie etwa 40 bis 90° C.

Der für die Kooxidation benötigte Sauerstoff kann in reiner Form oder in Form von Luft in den Reaktor eingeführt werden. Die Reaktion verläuft zwar, insbesondere in Gegenwart einer niederen Carbonsäure wie Essigsäure, unter Normaldruck, jedoch hat sich ein geringer Überdruck bis etwa 10 bar als günstig erwiesen. Besonders vorteilhaft ist eine Arbeitsweise, in der der Sauerstoffpartialdruck etwa 2 bar beträgt.

Die Oxidation des Zwischenproduktes $Z_1$ mit Sauerstoff oder Luft in Gegenwart von Salpetersäure zum Zwischenprodukt $Z_2$ in einer zweiten Reaktionsstufe c, gegebenenfalls in Gegenwart von Vanadinverbindungen, bringt den Vorteil, dass die in der ersten Stufe gebildete chinoide Zwischenverbindung $Z_1$ weiter zu $Z_2$ durchoxidiert wird und dadurch die für die Nachoxidation im Schritt $b_1$ benötigten Mengen an Chlor bzw. an Hypochlorit sowie an Alkali stark verringert werden können. Gleichzeitig erhöht sich die Ausbeute an Naphthalintetracarbonsäure.

In der zweiten Stufe c wird die durch Reaktion von Pyren mit den Perverbindungen entstandene dunkelbraune Lösung der Zwischenverbindung $Z_1$ in Essigsäure durch Einleiten von Sauerstoff oder gegebenenfalls von Luft in Gegenwart von Salpetersäure und gegebenenfalls Vanadinsalzen weiteroxidiert.

Für diesen Reaktionsschritt wird nach Beendigung der Percarbonsäure- oder Hydroperoxidzugabe zu der erhaltenen Lösung konzentrierte Salpetersäure in einer Menge von 0,1-10, vorzugsweise 0,5-3 mol, bezogen auf das eingesetzte Mol Pyren, und gegebenenfalls eine lösliche Vanadinverbindung in einer Menge von 0,0001-0,1 g, vorzugsweise 0,001-0,05 g-Atom, je Mol Pyren, zugegeben. Vorzugsweise wird die Vanadinverbindung als Ammoniumvanadat, das in konzentrierter Salpetersäure aufgelöst wurde, hinzugefügt.

Nach Erwärmen der essigsauren Lösung auf etwa 120 bis 180° C im Druckgefäss wird Sauerstoff oder Luft eingeleitet, so lange, bis die Reaktion abbricht, was durch Temperaturabfall infolge Nachlassens der Reaktionswärme oder bei Luftdosierung durch den Anstieg der Sauerstoffkonzentration im Abgas mittels eines Sauerstoffmessgerätes festgestellt werden kann.

Nach Beendigung des zweiten Oxidationsschrittes c kristallisiert beim Abkühlen aus dem eingeengten essigsauren Medium das braungefärbte Zwischenprodukt $Z_2$ aus, das sich leicht auf üblichen Wegen wie Abfiltrieren oder Zentrifugieren abtrennen lässt.

Die Oxidation der Zwischenprodukte $Z_1$ bzw. $Z_2$

mit Hypochlorit (Chlor) in der Reaktionsstufe $b_1$ bzw. $b_2$ erfolgt, indem die entstandene dunkelbraune Essigsäurelösung als solche nachoxidiert wird. Man kann aber auch vorher die Essigsäure ganz oder teilweise wiedergewinnen. Die zweite Möglichkeit ist die vorteilhaftere und kann in einem Dünnschicht- oder Fallfilmverdampfer ausgeführt werden. Die gewonnene Essigsäure wird als Lösungsmittel für den ersten Reaktionsschritt a wiederverwendet.

Die durch Abdestillieren der Essigsäure zurückbleibende Substanz wird bei etwa 40-70° C in wässerig-alkalischem Medium mit Chlor nachoxidiert.

Eine bevorzugte Ausführungsform besteht darin, dass in einem Reaktionskessel für die Nachoxidation von $Z_1$ etwa 10 mol wässerige Natron- bzw. Kalilauge und etwa 8 mol wässerige Natrium- oder Kaliumhypochloritlösung (sogenannte Chlorbleichlauge) je Mol anfangs eingesetztes Pyren vorgelegt wird. Für die Nachoxidation von $Z_2$ erniedrigt sich diese Menge auf etwa 6 mol Alkalilauge und etwa 4-5 mol Hypochlorit. Dann wird das Pyrenoxidat (Zwischenverbindung $Z_1$ bzw. $Z_2$) eingetragen und bei etwa 40-50° C gerührt. Dabei muss dafür Sorge getragen werden, dass durch Zugabe von Alkalilauge der pH-Wert nicht unter 10 absinkt. Nach einer Erhöhung der Temperatur auf etwa 60° C wird so lange Hypochloritlösung zugegeben, wie sie verbraucht wird. Der Endpunkt kann z.B. durch Tüpfeln auf Jodkalistärkepapier bestimmt werden. Die Lösung ist dann hellgelborange und kann gegebenenfalls durch Filtration geklärt werden. Sie enthält das Natrium- bzw. Kaliumsalz der Tetrasäure.

Die Hypochloritlösung kann auch im Oxidationskessel vor oder während der Reaktion durch Einleiten von Chlor in Alkalilauge hergestellt werden. Wenn genügend Alkali zur Bildung des Salzes der Tetrasäure vorhanden ist, kann durch Einleitung von Chlor in die wässerige Lösung, d.h. ohne Zusatz weiteren Alkalis, die Oxidation zufriedenstellend bewerkstelligt werden.

Die erhaltene Lösung des Alkalisalzes der Tetrasäure kann wie folgt zur freien Tetrasäure aufgearbeitet werden:

Überschüssiges Hypochlorit wird mit Natriumsulfit zerstört und dann bis zum pH-Wert von etwa 1 angesäuert. Hierbei wird als Säure wegen der ohnehin vorhandenen Chlorionen Salzsäure bevorzugt. Anschliessend wird die Mischung zum Sieden erhitzt. Die ausgefallene Tetrasäure wird abgesaugt, mit Wasser zur Entfernung der Salzsäure gewaschen und getrocknet. Das Produkt liegt dann zum überwiegenden Teil als Monoanhydrid der Tetrasäure vor.

*Beispiele*

*Beispiel 1*

(Vergleichsbeispiel gemäss SU-PS Nr. 202920, Beispiel 6)

Eine Mischung von 20,2 g (0,1 mol) Pyren und 200 ml Eisessig wurden in einem 250-ml-Rührkolben unter Rühren auf 75-80° C erhitzt. Dann wurde das Gemisch auf 30° C abgekühlt und mit 14,0 g Kobaltacetat/Tetrahydrat versetzt.

Zur Oxidation des Pyrens wurde innerhalb 18 h bei 30° C ein Ozon/Sauerstoff-Gemisch unter die Oberfläche der kräftig gerührten Suspension geleitet (2,1 g Ozon/h). Nach Beendigung der Oxidation wurde die tiefschwarz gefärbte Flüssigkeit auf 20° C gekühlt und filtriert. Ein geringer Niederschlag auf dem Filter wurde mit 3 ml Essigsäure gewaschen und getrocknet. Es wurden 0,5 g eines schwarzen Feststoffs erhalten.

Der schwarze Feststoff wurde nach Vorschrift des Beispiels 6 mit einer angepassten Hypochloritmenge oxidiert. Es wurden 0,3 g (1,1% d.Th.) Naphthalintetracarbonsäuremonoanhydrid isoliert.

Zur Kontrolle wurde das Filtrat des Ozon/Sauerstoff-Oxidats mit einer 4fachen Menge Wasser versetzt. Die ausgefallenen dunkelbraunen Flokken wurden abgesaugt und gewaschen. Der feuchte Filterkuchen wurde in einer Lösung von 50 g KOH in 500 ml Wasser suspendiert und mit 100 g einer 13%igen NaOCl-Lösung bis zur Aufhellung nachoxidiert, wie im Beispiel 6 (der SU-PS) beschrieben. Das Ansäuern mit Salzsäure ergab keine Fällung von Naphthalintetracarbonsäure.

*Beispiel 2*

12,12 g (0,06 mol) Pyren und 0,75 $Co(OCOCH_3)_2 \cdot 4\,H_2O$ wurden in einem Rührkolben in 100 ml Eisessig unter Rühren bei 110° C gelöst. Die Lösung wurde unter starkem Rühren auf 40° C abgekühlt, und zu der entstandenen Pyrensuspension liess man im Laufe von 10 min 81,1 g einer 19,5%igen Peressigsäure [15,82 g (0,208 mol) Peressigsäure in 65,3 g Essigsäure] zutropfen. Die Temperatur wurde durch Kühlung der Kolbenwand auf etwa 50° C gehalten. Die entstandene dunkelrote Lösung wurde im Rotationsverdampfer auf 50 ml eingeengt und in 200 ml Wasser eingegossen. Die ausgefallene dunkelbraune Masse wurde mit 200 ml Wasser gewaschen und dann in eine Lösung aus 40 g KOH und 300 g Chlorbleichlauge (Chlorgehalt 13% ≙ 0,55 mol Hypochlorit) unter Rühren eingetragen und auf etwa 50° C erwärmt. Nachdem alles Hypochlorit verbraucht war, wurden noch 200 g der Chlorbleichlauge zugefügt. Während der Oxidation wurde städig der pH-Wert kontrolliert und durch Zugabe von weiterem Kaliumhydroxid über 10 gehalten. Zum Schluss wurde auf 70° C erhitzt, überschüssiges Hypochlorit mit Natriumhydrogensulfit zerstört und mit konz. Salzsäure auf pH 1 angesäuert.

Die Naphthalintetracarbonsäure fiel in der Hitze als Monoanhydrid in Form kleiner Kristalle aus, die abgesaugt, mit Wasser gewaschen und bei etwa 70° C im Luftstrom getrocknet wurden.

Ausbeute: 8,8 g ≙ 51,2% d.Th. Naphthalintetracarbonsäure-1,4,5,8-monoanhydrid.

*Beispiel 3*

80,8 g (0,40 mol) Pyren und 5,0 g $Co(OCOCH_3)_2 \cdot 4\,H_2O$ wurden in einem Rührkolben in 650 ml Eisessig unter Rühren bei 110° C gelöst.

Die Lösung wurde unter starkem Rühren auf 40° C abgekühlt, und zu der entstandenen Pyrensuspension liess man im Laufe von 30 min 342,6 g einer 26,1%igen Peressigsäure [97,4 g (1,28 mol) Peressigsäure in 245,2 g Essigsäure] zutropfen. Die Temperatur wurde durch Kühlung der Kolbenwand auf etwa 50° C gehalten. Die entstandene dunkelrote Lösung wurde im Rotationsverdampfer auf 300 ml eingeengt und in 1000 ml Wasser eingegossen. Die ausgefallene dunkelbraune Masse wurde mit 1000 ml Wasser gewaschen und dann in eine Lösung aus 270 g KOH und 2000 g Chlorbleichlauge (Chlorgehalt 13% ≙ 3,67 mol Hypochlorit) unter Rühren eingetragen und auf etwa 50° C erwärmt. Nachdem alles Hypochlorit verbraucht war, wurden noch 1350 g der Chlorbleichlauge (2,48 mol Hypochlorit) zugefügt. Der Gesamtverbrauch an Hypochlorit lag bei 6,15 mol. Während der Oxidation wurde ständig der pH-Wert kontrolliert und durch Zugabe von weiterem Kaliumhydroxid über 10 gehalten. Zum Schluss wurde auf 70° C erhitzt, überschüssiges Hypochlorit mit Natriumhydrogensulfit zerstört und mit konz. Salzsäure auf pH 1 angesäuert.

Die Naphthalintetracarbonsäure fiel in der Hitze als Monoanhydrid in Form kleiner Kristalle aus, die abgesaugt, mit Wasser gewaschen und bei etwa 70° C im Luftstrom getrocknet wurden.

Ausbeute: 57,2 g ≙ 50,1% d.Th. Naphthalintetracarbonsäure-1,4,5,8-monoanhydrid.

*Beispiel 4*

In einen 1-l-Glasautoklaven wurden 60,6 g (0,3 mol) Pyren, die Lösung von 2,0 g (0,008 mol) Cobaltacetattetrahydrat in 250 g Eisessig und 220 g (5,0 mol) Acetaldehyd eingefüllt.

Unter einem Überdruck von 5 bar Stickstoff wurde auf etwa 70° C erhitzt und bei Erreichen dieser Temperatur Sauerstoff eingeleitet, wobei ein Abgasstrom von 15 l/h einreguliert wurde. Es setzte sofort eine exotherme Reaktion ein; die Temperatur wurde durch Kühlung des Autoklavenmantels auf 80-90° C gehalten. Nach 4 h war die Reaktion beendet. Von der braunen Reaktionslösung wurde die Essigsäure abdestilliert und der Rückstand (92,2 g) in eine Lösung aus 200 g KOH und 600 g Chlorbleichlauge (Chlorgehalt 13% ≙ 1,1 mol Hypochlorit) eingetragen. Die Lösung wurde auf etwa 50° C erwärmt und durch Zugabe von Natriumhypochloritlösung ständig oxidierend und durch Verbrauch von weiterer KOH oberhalb pH 10 gehalten. Nach Zugabe von etwa 0,5 kg Chlorbleichlauge wurde filtriert. Der Filterkuchen (22,7 g) bestand im wesentlichen aus nicht umgesetztem Pyren und wenig Kobaltoxid. Das dunkelrote Filtrat wurde durch Zugabe von weiterer Chlorbleichlauge bis zur Gelbfärbung aufgehellt. Zum Schluss wurde auf etwa 70° C erhitzt, überschüssiges Hypochlorit mit Natriumhydrogensulfit zerstört und mit konz. Salzsäure auf pH 1 angesäuert.

Die Naphthalintetracarbonsäure fiel in der Hitze als Monoanhydrid in Form kleiner Kristalle aus, die abgesaugt, mit Wasser gewaschen und bei etwa 70° C im Luftstrom getrocknet wurden.

Ausbeute: 27,4 g ≙ 32,0% d.Th. (bezogen auf eingesetztes Pyren) Naphthalintetracarbonsäure-1,4,5,8-monoanhydrid.

*Beispiel 5*

80,8 g (0,40 mol) Pyren wurden analog Beispiel 3 mit 97,4 g (1,28 mol) Peressigsäure umgesetzt. Die entstandene Reaktionslösung wurde einer weiteren Oxidation unter einer Sauerstoffatmosphäre unterworfen. Dazu wurde die Lösung in einem Rotationsverdampfer auf 370 g eingeengt und in einem Glasautoklaven unter 6 bar Überdruck im Sauerstoffstrom (25 l Abgas/h) auf etwa 130° C erwärmt. Im Laufe von 90 min wurde eine Lösung aus 0,6 g (0,005 mol) $NH_4VO_3$ und 37,8 g (0,60 mol) $HNO_3$ in 200 g Eisessig zudosiert. Die Reaktionstemperatur wurde zwischen 145 und 150° C gehalten.

Die abgekühlte Reaktionslösung wurde abgesaugt, der dunkelbraune Feststoff mit 60 ml Eisessig gewaschen und im Vakuum bei 50° C getrocknet (76 g). Die Filtrate wurden auf 150 g eingeengt und mit 150 ml Wasser versetzt. Der ausgefallene Feststoff wurde abgesaugt, mit Wasser gewaschen und getrocknet (16 g).

Die vereinigten Feststoffe (92 g) wurden im alkalischen Medium mit Hypochlorit nachoxidiert: Sie wurden in eine Lösung von 134 g (2,4 mol) KOH und 0,8 mol Natriumhypochlorit in 1 l Wasser bei 50 bis 60° C portionsweise eingetragen. Nachdem das vorgelegte Hypochlorit aufgebraucht war, wurde im Laufe von 4 h die wässerige Lösung von weiteren 0,8 mol Natriumhypochlorit zugefügt, so dass der Gesamtverbrauch 1,6 mol Hypochlorit betrug. Dabei trat eine Aufhellung zu Gelb auf. Von der Lösung wurden unlösliche Bestandteile abfiltriert, überschüssiges Natriumhypochlorit wurde mit Natriumbisulfit zerstört, und durch Zugabe von konz. Salzsäure wurde das Monoanhydrid der Naphthalintetracarbonsäure bei 60° C ausgefällt. Es wurde abgesaugt, mit Wasser gewaschen und bei etwa 70° C im Luftstrom getrocknet.

Ausbeute: 67,0 g ≙ 58,6% d.Th. Naphthalintetracarbonsäure-1,4,5,8-monoanhydrid.

*Beispiel 6*

Beispiel 5 wurde wiederholt, jedoch ohne den Zusatz von Ammoniumvanadat zur Salpetersäure in der zweiten Oxidationsstufe.

Ausbeute: 55,2 g ≙ 48,3% d.Th. Naphthalintetracarbonsäure-1,4,5,8-monoanhydrid.

## Patentansprüche

1. Verfahren zur Herstellung von Naphthalintetracarbonsäure-1,4,5,8 durch Oxidation von Pyren, dadurch gekennzeichnet, dass man Pyren, gelöst oder suspendiert in einer niederen Alkancarbonsäure, in Gegenwart einer katalytisch wirksamen Verbindung der Metalle Chrom, Mangan, Eisen, Kobalt, Nickel oder Kupfer mit einer organischen Perverbindung oder mit einem System in dem unter den Reaktionsbedingungen eine organische Perverbindung entsteht, zu einer Zwischenverbindung $Z_1$ oxidiert und diese in bekannter Weise mit Chlor (Hypochlorit) in wässerig-alkalischer Lösung in das Alkalisalz der Naphthalintetracarbonsäure-1,4,5,8 überführt.

2. Verfahren nach Anspruch 1, dadurch gekennzeichnet, dass man als niedere Alkancarbonsäure Essigsäure einsetzt.

3. Verfahren nach einem der Ansprüche 1 oder 2, dadurch gekennzeichnet, dass man als katalytisch wirksame Metallverbindung das Metallsalz einer niederen Alkancarbonsäure, vorzugsweise der Essigsäure, einsetzt.

4. Verfahren nach Anspruch 3, dadurch gekennzeichnet, dass man Cobalt- und/oder Manganacetat einsetzt.

5. Verfahren nach den Ansprüchen 1 bis 4, dadurch gekennzeichnet, dass man 0,005 bis 0,1 g-Atom der Metallverbindung je Mol Pyren einsetzt.

6. Verfahren nach den Ansprüchen 1 bis 5, dadurch gekennzeichnet, dass man als organische Perverbindung ein Hydroperoxid oder eine Percarbonsäure einsetzt.

7. Verfahren nach Anspruch 6, dadurch gekennzeichnet, dass man als Percarbonsäure Peressigsäure einsetzt.

8. Verfahren nach den Ansprüchen 1 bis 5, dadurch gekennzeichnet, dass man als System, in dem unter den Reaktionsbedingungen eine organische Perverbindung entsteht, Acetanhydrid und Wasserstoffperoxid einsetzt.

9. Verfahren nach den Ansprüchen 1 bis 5, dadurch gekennzeichnet, dass man als System, in dem unter den Reaktionsbedingungen eine organische Perverbindung entsteht, Aldehyde bzw. Ketone und Sauerstoff einsetzt.

10. Verfahren nach Anspruch 9, dadurch gekennzeichnet, dass man als Aldehyd Acetaldehyd einsetzt.

11. Verfahren nach den Ansprüchen 1 bis 10, dadurch gekennzeichnet, dass man die Zwischenverbindung $Z_1$ in Gegenwart von Salpetersäure mit Sauerstoff oder Luft zu einem Zwischenprodukt $Z_2$ oxidiert und erst dann die Umsetzung mit Chlor (Hypochlorit) in wässerig-alkalischer Lösung zum Alkalisalz der Naphthalintetracarbonsäure-1,4,5,8 durchführt.

12. Verfahren nach Anspruch 11, dadurch gekennzeichnet, dass man bei der Oxidation mit Sauerstoff oder Luft in Gegenwart von Salpetersäure eine Vanadinverbindung, vorzugsweise Ammoniumvanadat, einsetzt.

## Claims

1. Process for preparing naphthalene-1,4,5,8-tetracarboxylic acid by pyrene oxidation, characterized in that pyrene, dissolved or suspended in a lower alkanoic acid, is oxidized in the presence of a catalytically effective metal compound of chromium, manganese, iron, cobalt, nickel or copper by means of an organic peroxy compound or a system in which under the given reaction conditions an organic peroxy compound is formed, to yield an intermediate compound $Z_1$ and the said intermediate is converted in known manner using chlorine (hypochlorite) in an aqueous alkaline solution into the alkali salt of naphthalene-1,4,5,8-tetracarboxylic acid.

2. Process as claimed in Claim 1, wherein acetic acid is used as a lower alkanoic acid.

3. Process as claimed in Claim 1 or 2, wherein the metal salt of a lower alkanoic acid, preferably acetic acid, is used as a catalytically effective metal compound.

4. Process as claimed in Claim 3, wherein cobalt acetate and/or manganese acetate are used.

5. Process as claimed in Claims 1 to 4, wherein per mol of pyrene of from 0.005 to 0.1 gramme-atom of the metal compound is used.

6. Process as claimed in Claims 1 to 5, wherein a hydroperoxide or a percarboxylic acid is used as an organic peroxy compound.

7. Process as claimed in Claim 5, wherein peracetic acid is used as a percarboxylic acid.

8. Process as claimed in Claims 1 to 5, wherein acetic anhydride and hydrogen peroxide are used as a system in which under the given reaction conditions an organic peroxy compound is formed.

9. Process as claimed in Claims 1 to 5, wherein aldehydes or ketones in conjunction with oxygen are used as a system in which under the given reaction conditions an organic peroxy compound is formed.

10. Process as claimed in Claim 9, wherein acetaldehyde is used as an aldehyde.

11. Process as claimed in Claims 1 to 10, wherein the intermediate compound $Z_1$ is oxidized in the presence of nitric acid by means of oxygen or air to yield an intermediate compound $Z_2$, and only then the reaction using chlorine (hypochlorite) in an aqueous alkaline solution is carried out for converting the said intermediate $Z_2$ into the alkali salt of naphthalene-1,4,5,8-tetracarboxylic acid.

12. Process as claimed in Claim 11, wherein a vanadium compound, preferably ammonium vanadate, is used in the presence of nitric acid for the oxidation reaction by means of oxygen or air.

## Revendications

1. Procédé de préparation de l'acide naphtalènetétracarboxylique-1,4,5,8 par oxydation du pyrène, procédé caractérisé en ce qu'on oxyde le pyrène, dissous ou mis en suspension dans un acide alcanoïque inférieur, en présence d'un composé catalytiquement efficace d'un métal pris dans l'ensemble constitué par le chrome, le manganèse, le fer, le cobalt, le nickel et le cuivre, au moyen d'un percomposé organique ou d'un système dans lequel un percomposé organique se forme dans les conditions réactionnelles, oxydation qui conduit à un composé intermédiaire $Z_1$, et en ce qu'on transforme celui-ci, de manière connue, au moyen du chlore (hypochlorite), en solution aqueuse alcaline, en le sel de métal alcalin de l'acide naphtalènetétracarboxylique-1,4,5,8.

2. Procédé selon la revendication 1, caractérisé en ce qu'on utilise l'acide acétique comme acide alcanoïque inférieur.

3. Procédé selon l'une des revendications 1 ou 2, caractérisé en ce qu'on utilise, comme composé métallique à activité catalytique, un sel métallique d'un acide alcanoïque inférieur, de préférence de l'acide acétique.

4. Procédé selon la revendication 3, caractérisé en ce qu'on utilise l'acétate de cobalt et/ou l'acétate de manganèse.

5. Procédé selon l'une quelconque des revendications 1 à 4, caractérisé en ce qu'on met en jeu de 0,005 à 0,1 atome-gramme du composé métallique par mole de pyrène.

6. Procédé selon l'une quelconque des revendications 1 à 5, caractérisé en ce qu'on utilise, comme percomposé organique, un hydroperoxyde ou un acide percarboxylique.

7. Procédé selon la revendication 6, caractérisé en ce qu'on utilise l'acide peracétique comme acide percarboxylique.

8. Procédé selon l'une quelconque des revendications 1 à 5, caractérisé en ce qu'on utilise, comme système dans lequel un percomposé organique se forme dans les conditions de la réaction, l'anhydride acétique et le peroxyde d'hydrogène.

9. Procédé selon l'une quelconque des revendications 1 à 5, caractérisé en ce qu'on utilise, comme système dans lequel un percomposé organique se forme dans les conditions de la réaction, des aldéhydes ou des cétones et de l'oxygène.

10. Procédé selon la revendication 9, caractérisé en ce qu'on utilise l'acétaldéhyde comme aldéhyde.

11. Procédé selon l'une quelconque des revendications 1 à 10, caractérisé en ce qu'on oxyde le composé intermédiaire $Z_1$ en présence d'acide nitrique par de l'oxygène ou de l'air, oxydation qui conduit à un produit intermédiaire $Z_2$, et, seulement après, on effectue la réaction avec le chlore (hypochlorite) en solution aqueuse alcaline qui conduit au sel de métal alcalin de l'acide naphtalènetétracarboxylique-1,4,5,8.

12. Procédé selon la revendication 11, caractérisé en ce que, dans l'oxydation par l'oxygène ou l'air en présence d'acide nitrique, on utilise un composé du vanadium, de préférence le vanadate d'ammonium.